# EUROPEAN PATENT APPLICATION

(11) **EP 1 607 108 A1**
(43) Date of publication of application: **21.12.2005**
(21) Application number: 04722943.0
(22) Date of filing: 24.03.2004
(51) Int. Cl.: A61L 27/44, A61L 27/38, C12N 5/06

(54) **BLOOD VESSEL-SPECIFIC ORGANOGENESIS FROM EMBRYONIC STEM CELLS ON THREE-DIMENSIONAL MATRIGEL LAYER**

(30) Priority: 25.03.2003 JP 2003081875
(71) Applicant: Hayashi, Shinichiro, Kashiwara-shi, Osaka 5820006 (JP); AnGes MG, Inc., Ibaraki-shi, Osaka 567-0085 (JP)
(72) Inventor: HAYASHI, Shinichiro, Kashiwara-shi, Osaka 5820006 (JP)
(74) Representative: HOFFMANN EITLE
(86) International application number: PCT/JP2004/003996
(87) International publication number: WO 2004/084967

(57) **Abstract**

The present invention provides use of embryonic stem cells or embryonic stem cell-derived embryoid bodies cultured on growth factor-reduced Matrigel™, for treating diseases caused by ischemia or arteriosclerosis.

## Description

### Field of the Invention

The present invention relates to an agent for treating embryonic stem cells or embryonic stem cell-derived embrioid bodies.

### Prior Art

DE-A 19843234 disclosed heart cells differentiated from embryoid bodies produced by differentiation of embryonic stem or carcinoma cells, which are used as in vitro heart disease models.

### Disclosure of the Invention

The present invention relates to a method of using embryonic stem cells or embryonic stem cell-derived embrioid bodies cultured on growth factor-reduced Matrigel™, for treatment of diseases caused by ischemia or arteriosclerosis.

Also, the present invention relates to a method of treating ischemic disease or arteriosclerosis by angiogenesis, which comprises administering the above-mentioned embryonic stem cells or embryonic stem cell-derived embrioid bodies to a patient. Further, it relates to an agent for treating diseases caused by ischemia or arteriosclerosis, which comprises the above-mentioned embryonic stem cells or embryonic stem cell-derived embryoid bodies. Further, it relates to use of the above-mentioned embryonic stem cells or embryonic stem cell-derived embryoid bodies, for manufacturing an agent for treating diseases caused by ischemia or arteriosclerosis.

### Detailed Description of the Invention

Embryonic stem (ES) cells are self-replicable multipotent progenitor cells, and have a broad range of developmental potentials for various tissues.

Therefore, great attention is recently emerging into their therapeutic potentials for disease with high mortality.

Here, the present inventors demonstrated that ES cells could undergo vascular specific differentiation under the supports of extra cellular matrix (ECM) layers.

Murine ES cells were cultured as embrioid bodies (EBs) for two days, and then placed between two layers of growth factor-deprived matrigel with media containing 10%FBS.

After 5 days of culture in the matrigel layers without stimulation of angiogenesis-inducible factors, vessel sprouting was observed around EBs, and confirmed by pan-endothelial markers such as PECAM-1 or DiI-Acetylated LDL.

On day 10, sprouted vessels showed more differentiated morphology with branches. In contrast to the cells on the matrigel layers, EBs cultured on gelatin or fibronectin layers without growth factors did not show vascular specific differentiation around EBs, rather showed mixed populations of mixed several types of cells.

In conclusion, this model system could undergo vascular specific differentiation from ES cells.

These findings have important implication not only for testing the role of specific growth factors at the early stage of vascular development, but also for vascular specific tissue regeneration in cardiovascular diseases such as tissue ischemia and arteriosclerosis.

DE-19843234 has not disclosed any clinical use of embryonic stem cells.

The present invention indicated that EBs derived from ES cells could undergo vascular specific development under the supports of growth factor-deprived ECM components, matrigel. This system does not need specific growth factors, such as VEGF or FGF, for vasculogenesis and angiogenesis, although previous reports demonstrated that a limited population of ES cells which express an endothelial specific receptor, FLK, could develop vascular cells by stimulation of angiogenic growth factor, VEGF. The findings of the present inventors have important implication not only for testing the role of specific growth factors at the early stage of vascular development, but also for vascular specific tissue regeneration in cardiovascular diseases such as tissue ischemia and arteriosclerosis.

In the present invention, the embryonic stem cells or embryonic stem cell-derived embrioid bodies are preferably cultured between two layers of growth factor-reduced Matrigel™.

Further, the Matrigel™ is preferably selected from laminin, type4 collagen, entactin, and heparan-sulfated proteoglycan.

### Brief Description of the Drawings

Fig. 1A shows an embrioid body at day 1.
Fig. 1B shows an embrioid body on a growth factor-reduced matrigel at day 7.
Fig. 1C shows that an embrioid body is positive for the pan-endothelial marker.
Fig. 1D shows an embrioid body cultured on gelatin layers.
Fig. 1E shows an embrioid body cultured on fibronectin layers.
Fig. 2A shows an embrioid body cultured on one layer of matrigel.
Fig. 2B shows an embrioid body cultured between two layers of matrigel.
Fig. 2C is a high magnification view of Fig. 2B.

### Examples

The present invention is further illustrated by the following examples, but not limited thereto.

### Materials & Methods

### Cell culture

Undifferentiated murine embryonic stem (ES) cells were maintained in DMEM medium supplemented with 10%FCS, 1% non-essential amino acids, 1% 2-mercaptoethanol, and 0.1% leukemia inhibitory factor (LIF).

The ES cells were cultured with a hanging drop method (800 cells in each drops) for two days, and an embryoid body (EB) was developed from each drop.

Each EB was then cultured in LIF-deprived differentiation medium for another three days, and placed in a culture plate coated with a layer of growth factor-reduced matrigel (Becton Dickinson Labware), 1% gelatin, or fibronectin (Sigma).

For three-dimensional culture, the EBs were placed between two layers of matrigel.

Vessels sprouted from EBs were microscopically monitored everyday up to 7 days.

During the entire culture of the EBs, no angiogenic factor, such as VEGF or FGF, was added.

### Matrigel™

Matrigel™ is a soluble basement membrane gel, which can form a genuine reconstituted basement membrane at room temperature.

The major components of growth factor-reduced matrigel matrix are laminin, type 4 collagen, entactin, and heparan-sulfated proteoglycan.

In contrast to the standard matrigel, this matrigel matrix includes very tiny amounts of growth factors such as TGF-beta and IGF.

### Immunohistochemistry

Cultured EBs on matrigel layers were fixed in 2% paraformaldehyde at 4°C, and stained with following antibodies.

Primary antibody was rat anti-mouse platelet endothelial cell adhesion molecule-1 (PECAM-1)(clone MEC13.3, Pharmingen).

Secondary antibody was Alexsa 488-conjugated goat anti-rat IgG (Molecular Probe).

### Results

The results in Examples of the present invention are shown in Figs. 1 and 2. Fig. 1 shows data of three independent experiments (N=8) and indicates that EBs on matrigel showed vascular formation. Fig. 2 shows data of three independent experiments (N=8) and three layers of matrigel emphasized by the vascular formation of EBs.

ES cell-derived EBs on matrigel showed vascular formation.

ES cell-derived EBs could differentiate into cellular derivatives of all three primary germ layers of endodermal, ectodermal and mesodermal origin.

Therefore, there are mixed populations of differentiating cells in EBs.

Interestingly, EBs cultured on growth factor-reduced matrigel showed tree-like vascular formation around the edge of EBs on day 7 (Fig. 1B), although such a formation was not observed in EBs on day 1 (Fig. 1A).

On day 5, immunofluorescence staining of EBs revealed that these vessels like structures from EBs were positive for the pan-endothelial marker, PECAM-1 (Fig. 1C).

EBs on gelatin or fibronectin layer failed to show vascular formation.

In contrast to on matrigel layers, EBs cultured on gelatin layers (Fig. 1D) or fibronectin layers (Fig. 1E) failed to show vascular formation on day 7.

The three dimensional structures of EBs on these layers have been morphologically changed, and populations of mixed types of cells were growing on the cultured dishes.

Some colonies of cells were confirmed as beating cardiac myocytes.

Three dimensional layers of matrigel enhanced vascular formation from EBs.

The present inventors also examined whether three-dimensional layers of matrigel have an effect on vascular formation from EBs.

In contrast to EBs on one layer of matrigel (Fig. 2A), EBs cultured between two layers of matrigel showed more clear structure of vessels with branching and enough endothelium (Fig. 2B and C).

Fig. 2C is high magnification view of Fig. 2B.

### Conclusion

These series of observation indicated that ES cell-derived EBs could undergo vascular specific development under the supports of growth factor-deprived ECM components, matrigel.

This system do not need specific growth factors, such as VEGF or FGF, for vasculogenesis and angiogenesis, although previous reports demonstrated that a limited population of ES cells which expressed an endothelial specific receptor, FLK, could develop vascular cells by stimulation of angiogenic factor, VEGF.

The findings of the present inventors have important implication not only for testing the role of specific growth factors at the early stage of vascular development, but also for vascular specific tissue regeneration in cardiovascular diseases such as tissue ischemia and arteriosclerosis.

## Claims

1. A method of using embryonic stem cells or embryonic stem cell-derived embrioid bodies cultured on growth factor-reduced Matrigel™, for treatment of diseases caused by ischemia or arteriosclerosis.

2. The method according to claim 1, wherein the embryonic stem cells or embryonic stem cell-derived embrioid bodies have been cultured between two layers of growth factor-reduced Matrigel™.

3. The method according to claim 1 or 2, wherein the Matrigel™ is selected from laminin, type 4 collagen, entactin, and heparan-sulfated proteoglycan.

4. A method of treating ischemic diseases or arteriosclerosis by angiogenesis, comprising administering embryonic stem cells or embryonic stem cell-derived embrioid bodies cultured on growth factor-reduced Matrigel™, to a patient.

5. The method according to claim 4, wherein the embryonic stem cells or embryonic stem cell-derived embrioid bodies have been cultured between two layers of growth factor-reduced Matrigel™.

6. The method according to claim 4 or 5, wherein the Matrigel™ is selected from laminin, type 4 collagen, entactin, and heparan-sulfated proteoglycan.

7. An agent for treating diseases caused by ischemia or arteriosclerosis, which comprises embryonic stem cells or embryonic stem cell-derived embryoid bodies cultured on growth factor-reduced Matrigel™.

8. Use of embryonic stem cells or embryonic stem cell-derived embryoid bodies cultured on growth factor-reduced Matrigel™, for producing an agent for treating diseases caused by ischemia or arteriosclerosis.
